# EUROPEAN PATENT SPECIFICATION

(11) **EP 2 435 582 B1**
(45) Date of publication and mention of the grant of the patent: **23.10.2013**
(21) Application number: 10723980.8
(22) Date of filing: 24.05.2010
(51) Int. Cl.: C12Q 1/68

(54) **miRNA BIOMARKERS FOR THE DIAGNOSIS OF MUSCULAR DYSTROPHY AND ITS PROGRESSION**
miRNA BIOMARKERS FÜR DIE DIAGNOSE UND WEITERENTWICKLUNG DER MUSKELDYSTROPHIE
miRNA BIOMARKERS POUR LE DIAGNOSTIC ET LE DÉVELOPPEMENT DE LA DYSTROPHIE MUSCULAIRE

(30) Priority: 25.05.2009 EP 09161038
(43) Date of publication of application: 04.04.2012
(73) Proprietor: Università degli Studi di Roma "La Sapienza", 00185 Roma (IT)
(72) Inventor: BOZZONI, Irene, I-00185 Rome (IT); MARTONE, Julie, I-00185 Rome (IT); CACCHIARELLI, Davide, I-00185 Rome (IT); GIRARDI, Erika, I-00185 Rome (IT)
(74) Representative: Capasso, Olga
(86) International application number: PCT/EP2010/057088
(87) International publication number: WO 2010/136415

(56) References cited:
- WO-A-03/095647
- YUASA KATSUTOSHI ET AL: "MicroRNA-206 Is Highly Expressed in Newly Formed Muscle Fibers: Implications Regarding Potential for Muscle Regeneration and Maturation in Muscular Dystrophy" CELL STRUCTURE AND FUNCTION, vol. 33, no. 2, 2008, pages 163-169, XP002532415 ISSN: 0386-7196
- EISENBERG IRIS ET AL: "Distinctive patterns of microRNA expression in primary muscular disorders" PROCEEDINGS OF THE NATIONAL ACADEMY OF SCIENCES OF THE UNITED STATES OF AMERICA, vol. 104, no. 43, October 2007 (2007-10), pages 17016-17021, XP002532416 ISSN: 0027-8424
- MCCARTHY JOHN J ET AL: "MicroRNA-206 is overexpressed in the diaphragm but not the hindlimb muscle of mdx mouse" AMERICAN JOURNAL OF PHYSIOLOGY - CELL PHYSIOLOGY, vol. 293, no. 1, July 2007 (2007-07), pages C451-C457, XP002532417 ISSN: 0363-6143
- CALLIS THOMAS E ET AL: "Muscling through the microRNA world." EXPERIMENTAL BIOLOGY AND MEDICINE (MAYWOOD, N.J.) FEB 2008, vol. 233, no. 2, February 2008 (2008-02), pages 131-138, XP002532418 ISSN: 1535-3702
- DENTI MICHELA ALESSANDRA ET AL: "Chimeric adeno-associated virus/antisense U1 small nuclear RNA effectively rescues dystrophin synthesis and muscle function by local treatment of mdx mice" HUMAN GENE THERAPY, MARY ANN LIEBERT, NEW YORK ,NY, US, vol. 17, no. 5, 1 May 2006 (2006-05-01), pages 565-574, XP002493657 ISSN: 1043-0342
- DEUTEKOM VAN J C T ET AL: "Antisense-induced exon skipping restores dystrophin expression in DMD patient derived muscle cells" HUMAN MOLECULAR GENETICS, OXFORD UNIVERSITY PRESS, SURREY, vol. 10, no. 15, 1 January 2001 (2001-01-01), pages 1547-1554, XP002250908 ISSN: 0964-6906
- TOWNLEY-TILSON W H DAVIN ET AL: "MicroRNAs 1, 133, and 206: Critical factors of skeletal and cardiac muscle development, function, and disease" INTERNATIONAL JOURNAL OF BIOCHEMISTRY & CELL BIOLOGY, vol. 42, no. 8, 14 March 2009 (2009-03-14) , pages 1252-1255, XP002601173 ISSN: 1357-2725
- GRECO SIMONA ET AL: "Common micro-RNA signature in skeletal muscle damage and regeneration induced by Duchenne muscular dystrophy and acute ischemia" FASEB JOURNAL, vol. 23, no. 10, October 2009 (2009-10), pages 3335-3346, XP002601174 ISSN: 0892-6638

### SEQUENCE LISTING

<110> Università degli Studi di Roma "La Sapienza"
<120> miRNA BIOMARKERS FOR THE DIAGNOSIS OF DUCHENNE MUSCULAR DYSTROPHY, ITS PROGRESSION AND FOR MONITORING THERAPEUTIC INTERVENTIONS
<130> PCT109052
<160> 9
<170> PatentIn version 3.3
<210> 1
   <211> 22
   <212> RNA
   <213> Homo sapiens
<400> 1
   uggaauguaa agaaguaugu au 22
<210> 2
   <211> 22
   <212> RNA
   <213> Homo sapiens
<400> 2
   uggaauguaa ggaagugugu gg 22
<210> 3
   <211> 22
   <212> RNA
   <213> Homo sapiens
<400> 3
   ugucaguuug ucaaauaccc ca 22
<210> 4
   <211> 22
   <212> RNA
   <213> Homo sapiens
<400> 4
   uagcaccauc ugaaaucggu ua 22
<210> 5
   <211> 23
   <212> RNA
   <213> Homo sapiens
<400> 5
   uagcaccauu ugaaaucagu guu 23
<210> 6
   <211> 22
   <212> RNA
   <213> Homo sapiens
<400> 6
   uagcaccauu ugaaaucggu ua 22
<210> 7
   <211> 22
   <212> RNA
   <213> Homo sapiens
<400> 7
   uuuggucccc uucaaccagc ug 22
<210> 8
   <211> 22
   <212> RNA
   <213> Homo sapiens
<400> 8
   uuuggucccc uucaaccagc ua 22
<210> 9
   <211> 23
   <212> RNA
   <213> Homo sapiens
<400> 9
   uguaaacauc cuacacucuc agc 23

## Description

### Field of the invention

The invention refers to a method for the diagnosis of Duchenne Muscular Dystrophy (DMD) and related muscular degenerative disorders by means of serum or bioptic detection of a specific class of miRNAs.

### Background

Deletions and point mutations in the human 2.5 Mb-long dystrophin gene cause either the severe progressive myopathy Duchenne Muscular Dystrophy (DMD) or the milder Becker Muscular Dystrophy (BMD), depending on whether the translational reading frame is lost or maintained. In Duchenne Muscular Dystrophy, the complete absence of dystrophin leads to a dramatic decrease of the Dystrophin-Associated Protein Complex (DAPC) required to connect intracellular actin microfilaments to the extracellular matrix (Matsumura et al., 1994; Ervasti et al., 2008). As a consequence, muscle fibers become more sensitive to mechanical damage leading to muscle degeneration, chronic inflammatory response and increase in fibrosis, all of which exacerbate the dystrophic phenotype. All these traits are attenuated in Becker Dystrophy affected patients that have a very mild myopathic phenotype.

Making use of the exon skipping strategy the authors showed that it is possible to rescue dystrophin synthesis in human DMD myoblasts (De Angelis et al., 2002) as well as in Duchenne mice *(mdx)* (Denti et al., 2006). This treatment was demonstrated not only to rescue molecular parameters but also to provide a strong morpho-functional benefit to the muscles: in fact, histological examination of mice treated with exon skipping revealed a significant maintenance of muscle phenotype compared to *mdx* untreated littermates. In particular, whilst *mdx* mice showed massive inflammatory infiltration and degeneration, cured muscles displayed a correct tissue morphology and strong reduction in fibrosis (Denti et al., 2006). This effect was even more evident in old animals: *mdx* senile fibres showed a prominent reduction in the number of muscle with intensive myonecrosis, whereas in exon skipping-treated *mdx* mice preservation of muscle phenotype with a clear reduction in inflammation and fibrotic tissue was evident (Denti et al., 2008).

In Duchenne Muscular Dystrophy (DMD), dystrophin deficiency results also in dilated cardiomyopathy, which develops independently of pathological defects in skeletal muscle and represents a major cause of mortality and an important therapeutic target. In particular, although *mdx* mice rarely display cardiac abnormalities, they show myocardial necrosis and inflammation at senile age. The exon skipping treatment was shown to improve also the cardiac phenotype. While cardiac muscle of aged *mdx* mice showed large areas of fibrosis and mononuclear infiltration, the heart of systemically treated *mdx* mice resulted histologically preserved with significant reduction in fibrosis accumulation (Denti et al., 2008).

In subsequent work the authors discovered that dystrophin, besides its structural function, is also able to alter the expression pattern of a specific subset of miRNA genes relevant for muscle differentiation (Chen et al., 2006; Eisenberg et al., 2007) and proper tissue morphology (Van Rooij et al., 2008); they discovered that this regulation was mediated by a dystrophin-dependent pathway which affects the activity of the HDAC2 remodelling enzyme, thus suggesting a direct link between dystrophin and gene reprogramming through alteration of the epigenetic signature.

Moreover, the authors profiled the variations in the expression pattern of miRNAs in wild type versus DMD/*mdx* and exon skipping treated animals and found that specific miRNAs could become diagnostic for evaluating the damage state of the muscle tissue.

As part of the present inventions the authors have found that, as a consequence of muscle damage, specific muscle miRNAs are released into the serum and that their abundance is proportional to the extent of tissue damage.

Callis et al., Eisenberg et al., McCarthy et al., and Yuasa et al., all disclose miRNAs as markers for muscle dystrophic disorders in muscle samples

### Description of the invention

In this work, authors identified a specific signature of miRNAs (molecules known to play crucial functions in the differentiation commitment of several cell types and to be involved in many patho-physiological processes) that correlated with the DMD pathology. They described that a different miRNA expression profile exists between wild type and Duchenne cells (both human DMD and *mdx* mice). Furthermore, miRNA profile analysis in *cells* in which dystrophin has been rescued through the exon skipping approach indicated the existence of a specific class of miRNAs that are directly controlled by dystrophin: some of them being important for muscle differentiation and regeneration. A different group of miRNAs was found to change as a consequence of the benefit of the therapeutic treatment after dystrophin rescue; this class includes miRNAs diagnostic of the inflammatory and fibrotic processes.

Observed differences in the expression levels of both classes of miRNAs (dystromiR) can be utilized as biomarkers in order to evaluate the severity/progression of the disease in human patients as well as for measuring the outcomes of therapeutic interventions. In consideration of their link with muscle degeneration they propose that these miRNAs can be diagnostic also of other types of muscular disorders in which muscle fiber degeneration occurs with subsequent side effects such as inflammation and fibrosis.

Moreover, authors were able to measure alterations of the dystromiR profile directly in serum samples in a quantitative and rapid way.

Relevant dystro-miR are:
- **miR-223.** It is expressed in inflammatory cells and inflammation is known to be very relevant in Duchenne muscles. Upon dystrophin rescue the values are corrected to almost wild type levels due to the beneficial effect of dystrophin rescue on tissue integrity.
- **miR-29 and miR-30.** They are down-regulated in *mdx* muscles and this causes the increase in fibrosis.
- **miR-206.** It is expressed in activated satellite cells and its levels correlate with the amount of regenerating fibers. They increase in dystrophic muscles paralleling muscle damage. Its levels are also high after exon skipping, since under these conditions muscle regeneration is still very active. miR-206 levels increase in the serum of dystrophic mice and human DMD patients; they are rescued almost to wt levels in mice treated with exon skipping treatment.
- **miR-1 and miR-133.** They are markers of muscle differentiation. Their accumulation decreases in dystrophic muscles while it is restored upon dystrophin rescue. miR-1 levels increase in the serum of dystrophic mice and human DMD patients; they are rescued almost to wt levels in mice treated with exon skipping treatment.

It is an object of the instant invention a method for the diagnosis of a muscle disorder in which muscle fiber degeneration occurs in a subject consisting of *in vitro* determining whether:
- at least one molecule belonging to the following group: miR-206 and miR-1 or
- the miR-133 molecule
has increased levels in a blood or serum sample of the subject when compared to a sample obtained from a healthy individual. Another object of the present invention is a method for monitoring the progress of a therapeutic treatment of a muscle disorder in which muscle fiber degeneration occurs in an affected subject consisting of *in vitro* detecting:
- at least one molecule belonging to the following group: miR-206 and miR-1 or
- the miR-133 molecule
in a blood or serum sample of the subject, wherein the subject sample has increased levels of any one of said markers at the beginning of the treatment, compared to a sample from a control individual. Preferably the muscle disorder is Duchenne Muscular Dystrophy.
In a preferred embodiment of the method of the invention the molecules are miR-206 and miR-1.The detecting of at least one molecule belonging to the following group: miR-206
and miR-1, or of the miR-133 molecule is performed preferably by reverse amplification of said molecule and real time detection of amplified products.

### Figure legends

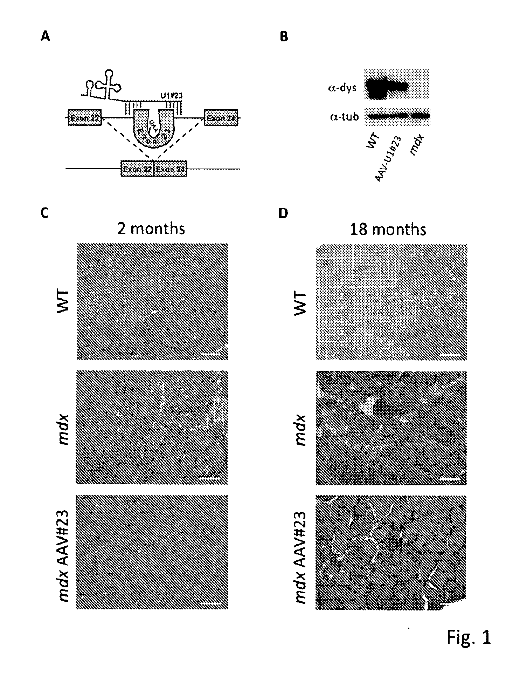

**Figure 1****. Analysis of muscle morphology in wild type, mdx and exon skipping treated animals.** (A) Schematic representation of the exon skipping strategy in the mdx mouse. (B) Western blot with anti-dystrophin (DYS) and anti-tubulin (TUB) antibodies performed on protein extracts from the gastrocnemius of WT, *mdx* and AAV#23-treated *mdx* animals sacrificed 4 weeks after systemic injection of AAV-U1#23 virus. Hematoxilin/Eosin (H&E) staining on WT, mdx and AAV#23-treated *mdx* analyzed after 4 weeks (C) or 18 months (D). Original magnification, X20. Scale bar is 100 µm.

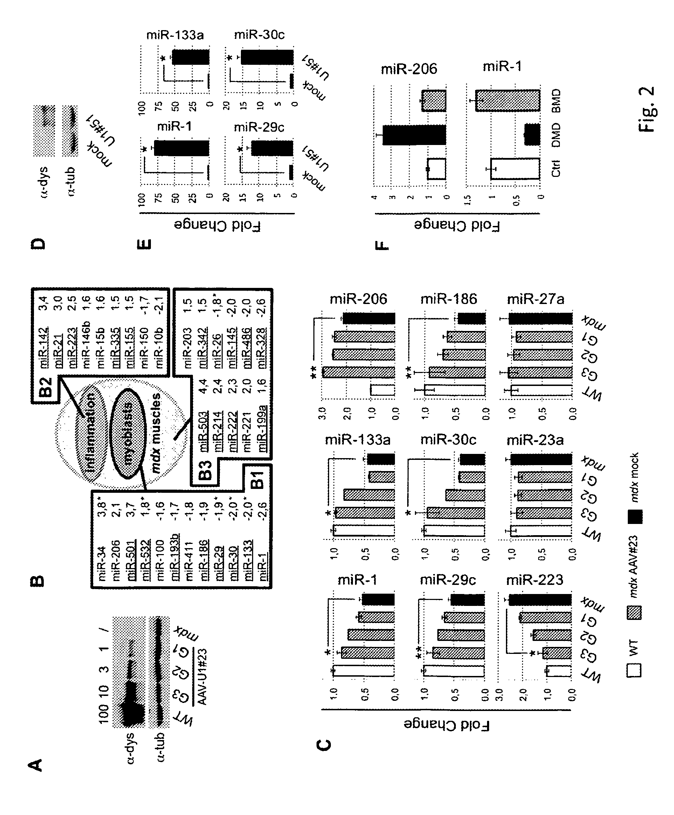

**Figure 2****. miRNA profiling** (A) Western blot with anti-dystrophin (DYS) and anti-tubulin (TUB) antibodies performed on protein extracts from the gastrocnemius of WT, *mdx* and AAV#23-treated *mdx* (G1, G2 and G3) animals sacrificed 4 weeks after systemic injection of AAV-U1#23 virus. (B) A list of differentially expressed miRNAs (at least 1,5 fold variation), in WT and *mdx* gastrocnemius, was derived from the values of real-time based low density arrays. In consideration of the cellular heterogeneity of the dystrophic muscle, we distinguished three miRNA groups (Box B1-B3). miRNAs, rescued towards WT levels in exon skipping-treated mice, are underlined. The asterisks indicate miRNA families. (C) Histograms show miRNA relative expression in WT, G1-G3 and *mdx* mice, measured by qRT-PCR. The invariant miR-23a and miR-27a were used as controls. Expression levels were normalized to snoRNA55 and shown with respect to WT set to a value of 1. (D) Western blot with anti-dystrophin (DYS) and anti-tubulin (TUB) antibodies on protein extracts from human DMD myoblasts infected with the LV#51 lentiviral vector (LV#51) or a control vector (LV-mock) and differentiated for 7 days. (E) miRNA levels in control (LV-mock) and antisense-treated (LV#51) DMD cells measured by qRT-PCR. U6 snRNA was used as endogenous control. Relative expression levels are shown with respect to mock-infected DMD cells, set to a value of 1. (F) Histograms show miRNA relative expression in healthy (Ctrl) versus Duchenne (DMD) and Becker (BMD) patients, measured by qRT-PCR. Expression levels were normalized to U6 snRNA and shown with respect to a healthy control set to a value of 1.

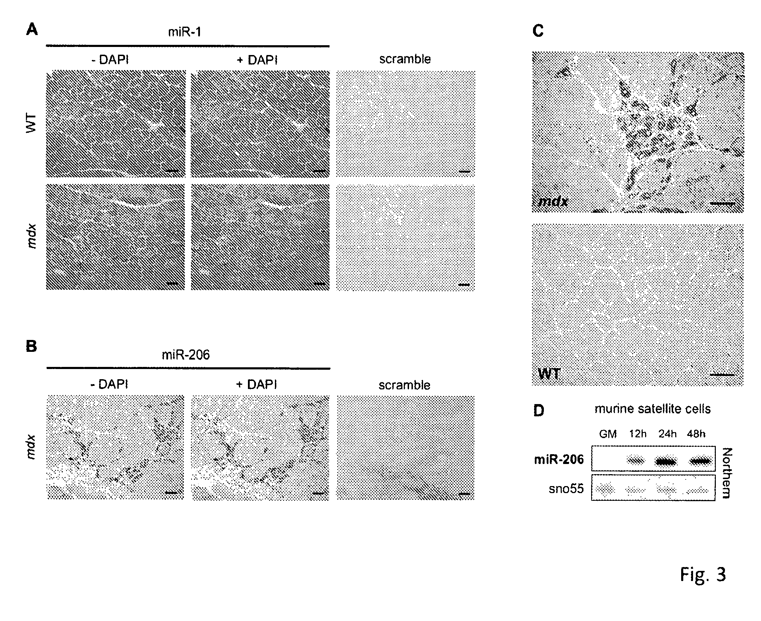

**Figure 3****. miR-206 expression** (A) A miR-1 DIG-labelled probe was hybridized on WT and *mdx* gastrocnemius sections. Original magnification, X20. Scale bar 100 µm. (B) A miR-206 DIG-labelled probe was hybridized on *mdx* gastrocnemius sections. DAPI staining is shown. Original magnification, X20. Scale bar 100 µm. (C) Same as in B) with original magnification, X40. Scale bar 50 µm. (D) Northern blot for miR-206 and snoRNA55 on RNA from proliferating satellite cells (GM - growth medium) and after shift to differentiation medium for the indicated hours.

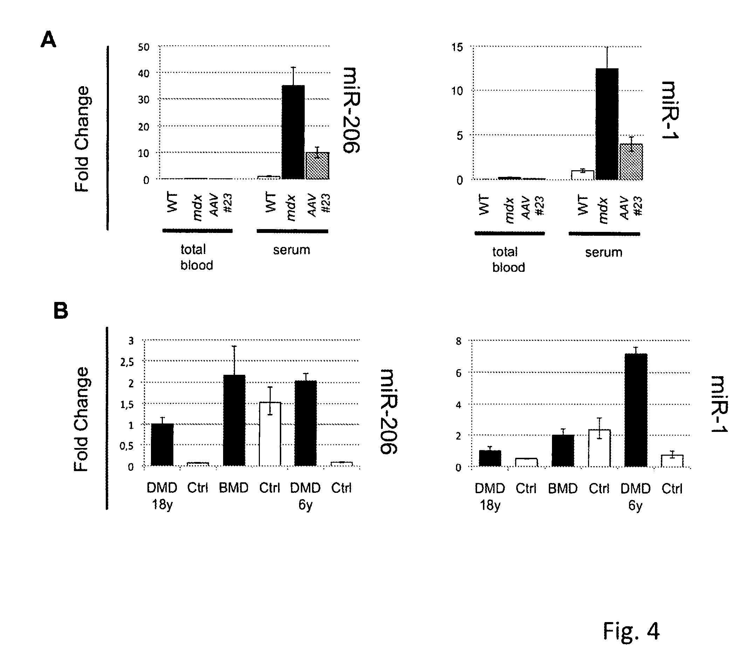

**Figure 4****. miRNA expression in blood and serum.** (A) Histograms show miR-206 and miR-1 relative expression in total blood and serum from WT (white bars) and *mdx* (black bars) and AAV#23 treated *mdx* (grey bar) mice, measured by qRT-PCR. Fold changes are shown with respect to WT serum levels set to a value of 1. (B) Histograms show miR-206 and miR-1 relative expression in serum from Duchenne (DMD) and Becker (BMD) patients of different ages compared with their healthy controls (Ctrl). Fold changes are shown with respect to WT serum levels set to a value of 1.

### Materials and Methods

### Sequence of miRNAs under analysis

Mature miRNAs as below show perfect sequence conservation between human and mouse. The mature sequence of the miRNA not listed can be found on the public database of miRNAs (www:mirbase.org).
miR-1: uggaauguaaagaaguauguau (SEQ ID No. 1, MI0000651) (the same mature miRNA sequence derives from the two miRNA genes (miR-1-1 and miR-1-2).
miR-206: uggaauguaaggaagugugugg (SEQ ID No. 2, MI0000490)
miR-223: ugucaguuugucaaauacccca (SEQ ID No. 3, MI0000300)
miR-29a: uagcaccaucugaaaucgguua (SEQ ID No. 4, MI0000087)
miR-29b1/2: uagcaccauuugaaaucaguguu (SEQ ID No. 5, MI0000105)
miR-29c: uagcaccauuugaaaucgguua (SEQ ID No. 6, MI0000735)

The miR-29 family includes miR-29a miR-29b1/2 and miR-29c (each produced from a specific locus). When miR-29 expression levels are indicated, the reported values are the mean result of all the three mature miRNAs. Also in this case the same mature miRNA sequence can derive from different genomic locations (miR-29b-1 and miR-29b-2 genes).
miR-133a1/2: uuugguccccuucaaccagcug (SEQ ID No. 7, MI0000450)
miR-133b: uuugguccccuucaaccagcua (SEQ ID No. 8, MI0000822)

The miR-133 family includes miR-133a1/2 and miR-133b (each produced from a specific locus). When miR-133 expression levels are indicated, the reported values are the mean result of the two mature miRNAs. Also in this case the same mature miRNA sequence can derive from different genomic locations (miR-133a-1 and miR-133a-2 genes).
miR-30c1/2: uguaaacauccuacacucucagc (SEQ ID No. 9, MI0000736)
Spiked miRNAs used as loading controls were ath-mir-159a (MI0000189), cel-mir-2 (MI0000004), cel-lin-4 (MI0000002).

**Animal treatments and constructs.** 6 week-old *mdx* mice were tail vein injected with 0.5-1x10¹² genome copies of the AAV-U1#23 (AAV#23) or virus as previously described (Denti et al., 2006) and sacrificed after 4 weeks.

**RNA preparation and analysis.** Total RNA was prepared from liquid nitrogen powdered tissues or cells homogenized in QIAZOL reagent (QIAGEN). miRNA profiling was performed as described below while analysis of individual miRNAs and mRNAs was performed using specific TaqMan (Applied Biosystems) or SYBRgreen (QIAGEN) assays. Relative quantification of individual miRNAs was performed using snoRNA55 or U6 snRNA.

Total RNA was extracted from 200-400 µl of human serum with miRNeasy (QIAGEN). RNA extraction was performed with or without spiked miRNA mimic (QIAGEN) (ath-mir-159a, cel-mir-2 e cel-lin-4) added to qiazol reagent (QIAGEN) before extraction. RNA retrotranscription and miRNA quantification was performed with both Taqman (Applied Biosystems) and SYBRgreen (mirscript QIAGEN) systems according manufacturers specifications. Relative quantification was performed using healty controls as reference samples and spiked miRNA to normalized the amount of starting material and cDNA used in real time analysis.
miRNA assays ID used in real time analyses were:

### QIAGEN:

Hs_miR-223_1 (MS00003871)
Hs_miR-206_1 (MS00003787)
Hs_miR-1_1 (MS00008358)
Hs_miR-29c_1 (MS00003269)
Hs_miR-133b_1 (MS00007385)
Hs_miR-133a_1 (MS00007378)
Hs_miR-29b_1 (MS00006566)
Hs_miR-29a_1 (MS00003262)
Hs_miR-30c_2 (MS00009366)
ath-mir-159a (ath-mir-159a_9)
cel-mir-2 (cel-mir-2_5)
cel-lin-4 (cel-lin-4_3)

### APPLIED BIOSYSTEMS:

Hs_miR-223 (002295)
Hs_miR-206 (000510)
Hs_miR-1 (002222)
Hs_miR-29c (000587)
Hs_miR-133b (002247)
Hs_miR-133a (002246)
Hs_miR-29b (000413)
Hs_miR-29a (002112)
Hs_miR-30c (000419)
ath-mir-159a (000338)
cel-mir-2 (000195)
cel-lin-4 (000258)

**miRNA profiling and data analysis.** To synthesize single-stranded cDNA, 700 ng of total RNA were reverse transcribed using the miRNA reverse transcription kit in combination with the stem-loop Megaplex RT primers pool A (Applied Biosystem). 335 small RNAs were profiled using the Applied Biosystems TaqMan Low Density Array. Since instrument and liquid handling variations were shown to be minimal, no PCR replicates were measured according to manufacturer specifications. Raw Ct values were calculated using the SDS software V.2.3 and data were subsequently analyzed through StatMiner platform according to the manufacturer pipeline. StatMiner Genorm algorithm was applied to determine the best invariant endogenous controls on a list of 5.

**Protein and miRNA in situ analyses.** Western blot on total extracts, H&E staining and *in situ* analyses on 7 µm-thick gastrocnemius cryosections were performed as described (Denti et al., 2006). miRNA in situ hybridization was performed in formaldehyde and EDC-fixed gastrocnemius cryosections according to Pena et al. (2009).

**Statistical analyses.** Each data shown in qRT-PCR is the result of at least three indipendent experiments performed on at least three different samples/animals. Data are shown as mean ± standard deviation. Unless specifically stated, statistical significance of differences between means was assessed by two-tailed t-test and a p < 0.05 was considered significant.

### Results

6-week old *mdx* animals were tail vein injected with AAV recombinant viruses (AAV-U1#23) carrying a U1-chimeric antisense construct (Fig.1A) previously reported to induce the skipping of the mutated exon 23 of the murine dystrophin gene and to restore dystrophin synthesis (Denti et al., 2006 and 2008). After 1 month, *mdx* and treated-*mdx* seeblings were sacrificed in parallel with wild type (WT) isogenic/aged matched animals. Different muscular districts were dissected and subdivided for RNA and protein analysis. Dystrophin rescue was obtained (Denti et al., 2006 and Fig.1B-D). We took advantage of the intrinsic variability of *in vivo* transduction, following systemic delivery (Denti et al., 2006), in order to classify the animals on the basis of dystrophin rescue levels. Three different groups, each one including at least three different individuals, were obtained (G1, G2 and G3); compared to wild type mice, they display ≤1%, 1-5% and 5-10% of dystrophin rescue, respectively (Fig.2A). Even if these levels seem very low to confer any beneficial effect, it was demonstrated that amounts of dystrophin as low as a few percent are able to confer long life benefit to *mdx* muscles (Denti et al., 2008; Ghahramani Seno et al., 2008). Dystrophin rescue paralleled the morphological amelioration of the transduced muscle (Fig 1C).

Real-time based low density arrays were performed on RNA from the gastrocnemius of wild type, *mdx,* and AAV-U1#23-treated *mdx* animals. miRNA expression levels, normalized for 5 endogenous controls, revealed clear differences between WT and *mdx.* In the diagram of Fig. 2B, the miRNAs displaying the most significant variations (fold-change > 1,5) between WT and *mdx* are reported. Aware of the cellular heterogeneity of the dystrophic muscle, a parallel profile analysis on human DMD myoblasts was performed. Relevant differences between the animal and the *in vitro* cultured differentiated myoblasts were due to miRNAs belonging to cell types other than muscle ones (box B2 and B3) and, in particular as expected for a damaged dystrophic fiber, to miRNAs related to the inflammatory process (box B2, Fazi et al., 2005; Baltimore et al., 2008). Among the others, the B1 group contains miRNAs in common between cultured myoblasts and dissected muscles. The underlined miRNAs identify those species that in AAV-U1#23-treated *mdx* are recovered to levels similar to WT. Interestingly, this effect was proportional to the levels of dystrophin rescue, with G1 animals showing a very limited effect and G3 providing levels very close to the wild type ones. Individual qRT-PCR were performed on B1 miRNAs and on a selection of control miRNAs. miR-1, miR-133a, miR-186, miR-29c and miR-30c, showing reduced levels in *mdx,* recover towards wild type levels in the three groups of treated animals proportionally to the level of dystrohin rescue (Fig. 2C). At variance with miR-1 and miR-133 myomiRs, miR-206 levels increase in *mdx* and in treated mice. Interestingly, miR-29 and miR-30 inversely correlate with the fibrotic state of the muscle. Indeed they have been shown to repress the translation of mRNAs for proteins of the extracellular matrix (van Rooij et al., 2008).

Interestingly, the inflammatory-specific miR-223 (Fazi et al., 2005), very abundant in *mdx,* is proportionally reduced in the three different groups of animals, indicating the amelioration of the inflammatory state of the muscle due to dystrophin rescue (see fig.1B-D and Denti et al., 2008). The ubiquitous miR-23a and 27a, unchanged in WT and *mdx,* behaved similarly also in treated animals. The relative expression of the selected miRNAs in WT, *mdx* and G3 animals was the same also in other muscle districts (not shown), demonstrating that the miRNA expression profile correlated with dystrophin rescue in a body-wide manner.

These data indicate that a specific subset of miRNAs undergoes altered expression in Duchenne Muscular Dystrophy as a direct consequence of dystrophin absence, while a different subset varies as a consequence of fiber damage.

These experiments clarify a new link between dystrophin and important genes encoding for miRNAs acting as regulators of muscle tissue differentiation and morphology. Moreover, the altered pattern of miRNA expression in DMD can be extended to other muscle disorders in which the DAPC complex is absent or altered (such as in Limb-girdle muscular dystrophy). These data also support the hypothesis that the morpho-functional benefit observed in exon-skipping *mdx* treated animals (Denti et al., 2008) may be due not only to the structural amelioration of the muscle membrane but also to an intracellular cascade of events controlling the expression of genes relevant for proper muscle homeostasis.

Among miRNAs that vary as a consequence of fiber damage the myomiR-206 was selected for further investigations. *In situ* hybridization analyses were performed on WT and *mdx* gastrocnemius muscles using DIG-labelled probes: increased levels of miR-206 in newly formed muscle fibers/myotubes were found (Figure 3B and 3C). Signals for miR-206 were restricted to *mdx* and only in immature regenerating fibers with centralized nuclei whereas the miR-1 probe, showed intense accumulation in mature differentiated fibers in both WT and *mdx* fibers (Figure 3A). Therefore, increased expression of miR-206 in *mdx* muscles is due to differentiating satellite cells (Figure 3D). These data suggest a role of miR-206 in active regeneration and efficient maturation of skeletal muscle fibers.

Finally, the possibility that, as a consequence of muscle damage, specific muscle miRNAs could be released into the blood was tested. qRT-PCR on blood and serum of wild type versus dystrophic animals was performed. Fig.4A indicates that miR-206 and miR-1 are found at much higher levels (30 and 15-fold increase respectively) in the serum of *mdx* animals with respect to wild type ones. In animals treated with exon skipping normal levels of dystromirs were detected also in the blood, then authors prove that miRNA measurement can be utilized also during DMD therapeutic intervention in order to follow the benefit of the treatment. miR-206 and miR-1 quantification was performed also on human serum of Duchenne and Becker boys. Increase of both myomiRs is specifically detected in Duchenne patients with respect to their healthy brothers/sisters. Moreover, the myomiRs levels are higher in young patients (6 year old) with respect to older ones (18 year old) consistent with the higher levels of degeneration/regeneration observed in the first decade of life of these patients.

These results indicate that, as a consequence of muscle damage, miRNAs specifically expressed in these cells are released into the blood in which they are accumulated in a stable form and proportionally to the extent of damage. Therefore, this feature can be utilized as a simple and non invasive way for evaluating the level of muscle damage. Interestingly, the same approach can be extended to other diseases in which muscle damage is a primary cause or a secondary effect.

### References

- Baltimore, D., et al. MicroRNAs: new regulators of immune cell development and function. Nat. Immunol. 9:839-45 (2008).
CALLIS THOMAS E ET AL: "Muscling through the microRNA world." EXPERIMENTAL BIOLOGY AND MEDICINE (MAYWOOD, N.J.) FEB 2008, vol. 233, no. 2, February 2008 (2008-02), pages 131-138, XP002532418 ISSN: 1535-3702
- Chen J.F. et al. (2006) The role of mic/roRNA-1 and microRNA-133 in skeletal muscle proliferation and differentiation. Nat Genet. 38:228-33.
- De Angelis F., et al. (2002) Chimeric snRNA molecules carrying antisense sequences against the splice junctions of exon 51 of the dystrophin pre-mRNA induce exon skipping and restoration of a corrected phenotype in Δ48-50 DMD cells, Proc. Natl. Acad. Sci. USA, 99:9456-61.
- Denti, M.A., et al. Body-wide gene therapy of Duchenne Muscular Dystrophy in the mdx mouse model. Proc. Natl. Acad. Sci. USA 103:3758-3763 (2006).
- Denti M. A., et al. Life-long benefit of AAV/antisense-mediated exon skipping in dystrophic mice. Hum. Gene Ther-. 19:601-608 (2008).
- Eisenberg, I., et al. Distinctive patterns of microRNA expression in primary muscular disorders. Proc. Natl. Acad. Sci. USA 104:17016-17021 (2007).
- Ervasti, J.M., Sonnemann, K.J. Biology of the striated muscle dystrophin-glycoprotein complex. Int Rev Cytol. 265:191-225 (2008).
- Fazi, F., et al. A mini-circuitry comprising microRNA-223 and transcription factors NFI-A and C/EBPα regulates human granulopoiesis. Cell 123:819-831 (2005).
- Ghahramani Seno, M.M., et al. RNAi-mediated knockdown of dystrophin expression in adult mice does not lead to overt muscular dystrophy pathology. Hum. Mol. Genet. 17:2622-2632 (2008).
- Lee, Y., et al. MicroRNA genes are transcribed by RNA polymerase II. EMBO J. 23: 4051-4060(2004)
- Matsumura, K., et al. Expression of dystrophin-associated proteins in dystrophin-positive muscle fibers (revertants) in Duchenne muscular dystrophy. Neuromuscul. Disord. 4:115-120 (1994).
MCCARTHY JOHN J ET AL: "MicroRNA-206 is overexpressed in the diaphragm but not the hindlimb muscle of mdx mouse" AMERICAN JOURNAL OF PHYSIOLOGY - CELL PHYSIOLOGY, vol. 293, no. 1, July 2007 (2007-07), pages C451-C457, XP002532417 ISSN: 0363-6143
- Van Rooij, E., et al. Dysregulation of microRNAs after myocardial infarction reveals a role of miR-29 in cardiac fibrosis. Proc. Natl. Acad. Sci. USA 105:13027-13032 (2008).
YUASA KATSUTOSHI ET AL: "MicroRNA-206 Is Highly Expressed in Newly Formed Muscle Fibers: Implications Regarding Potential for Muscle Regeneration and Maturation in Muscular Dystrophy" CELL STRUCTURE AND FUNCTION, vol. 33, no. 2, 2008, pages 163-169, XP002532415 ISSN: 0386-7196

## Claims

1. A method for the diagnosis of a muscle disorder in which muscle fiber degeneration occurs in a subject consisting of *in vitro* determining whether:
- at least one molecule belonging to the following group: miR-206 and miR-1, or
- the miR-133 molecule
has increased levels in a blood or serum sample of the subject when compared to a sample obtained from a healthy individual.

2. A method for monitoring the progress of a therapeutic treatment of a muscle disorder in which muscle fiber degeneration occurs in an affected subject consisting of *in vitro* detecting:
- at least one molecule belonging to the following group: miR-206 and miR-1, or
- the miR-133 molecule
in a blood or serum sample of the subject,
wherein the subject sample has increased levels of any one of said markers at the beginning of the treatment, compared to a sample from a control individual.

3. The method according to claim 1 or 2 wherein said muscle disorder is Duchenne Muscular Dystrophy.

4. The method according to claim 1 or 2 wherein the molecules are miR-206 and miR-1.

5. The method according to any of previous claims wherein the detecting of at least one molecule is performed by reverse amplification of said molecule and real time detection of amplified products.

## Patentansprüche

1. Verfahren für die Diagnose einer Muskelerkrankung, bei der eine Muskelfaserdegeneration stattfindet, in einem Individuum, bestehend aus dem Bestimmen *in vitro,* ob:
- mindestens ein Molekül, das zu der folgenden Gruppe gehört: miR-206 und miR-1, oder
- das miR-133-Molekül
erhöhte Spiegel in einer Blut- oder Serumprobe des Individuums verglichen mit einer von einem gesunden Individuum erhaltenen Probe aufweist.

2. Verfahren zum Überwachen des Fortschritts einer therapeutischen Behandlung einer Muskelerkrankung, bei der eine Muskelfaserdegeneration stattfindet, in einem betroffenen Individuum, bestehend aus dem Detektieren *in vitro:*
- mindestens eines Moleküls, das zu der folgenden Gruppe gehört: miR-206 und miR-1, oder
- des miR-133-Moleküls
in einer Blut- oder Serumprobe des Individuums,
wobei die Individuumprobe verglichen mit einer Probe von einem Kontrollindividuum zu Beginn der Behandlung erhöhte Spiegel eines beliebigen der Marker aufweist.

3. Verfahren nach Anspruch 1 oder 2, wobei es sich bei der Muskelerkrankung um Muskeldystrophie des Typs Duchenne handelt.

4. Verfahren nach Anspruch 1 oder 2, wobei es sich bei den Molekülen um miR-206 und miR-1 handelt.

5. Verfahren nach einem der vorherigen Ansprüche, wobei das Detektieren mindestens eines Moleküls durch reverse Amplifikation des Moleküls und durch Echtzeit-Detektion amplifizierter Produkte durchgeführt wird.

## Revendications

1. Procédé de diagnostic d'un trouble musculaire dans lequel se produit une dégénérescence des fibres musculaires chez un sujet, ledit procédé consistant à déterminer *in vitro* si :
- au moins une molécule appartenant au groupe suivant : miR-206 et miR-1, ou
- la molécule miR-133
présente des niveaux accrus dans un échantillon de sang ou de sérum du sujet comparé à un échantillon prélevé sur un individu sain.

2. Procédé de surveillance de l'évolution d'un traitement thérapeutique d'un trouble musculaire dans lequel se produit une dégénérescence des fibres musculaires chez un sujet affecté, ledit procédé consistant en la détection *in vitro* de :
- au moins une molécule appartenant au groupe suivant : miR-206 et miR-1, ou
- la molécule miR-133
dans un échantillon de sang ou de sérum du sujet,
dans lequel l'échantillon du sujet a des niveaux accrus de l'un quelconque desdits marqueurs au début du traitement, comparé à un échantillon d'un individu témoin.

3. Procédé selon la revendication 1 ou 2, dans lequel ledit trouble musculaire est la dystrophie musculaire de Duchenne.

4. Procédé selon la revendication 1 ou 2, dans lequel les molécules sont miR-206 et miR-1.

5. Procédé selon l'une quelconque des revendications précédentes, dans lequel la détection d'au moins une molécule est réalisée par amplification inverse de ladite molécule et par détection en temps réel des produits amplifiés.
